# EUROPEAN PATENT SPECIFICATION

(11) **EP 2 543 348 B1**
(45) Date of publication and mention of the grant of the patent: **18.03.2020**
(21) Application number: 12187487.9
(22) Date of filing: 20.11.2002
(51) Int. Cl.: A61F 7/02, A61F 7/00

(54) **IMPROVED MEDICAL THERMAL ENERGY EXCHANGE PAD**
VERBESSERTE MEDIZINISCHE WÄRMETAUSCHAUFLAGE
COMPRESSE MÉDICALE D'ÉCHANGE THERMIQUE AMÉLIORÉE

(30) Priority: 27.02.2002 US 87533; 27.02.2002 US 87534
(43) Date of publication of application: 09.01.2013
(62) Divisional of application: 02806905.2
(73) Proprietor: Medivance Incorporated, Louisville, CO 80027 (US)
(72) Inventor: Hoglund, Michael, R., Mead, CO Colorado 80542 (US); Carson, Gary, A., Golden, CO Colorado 80401 (US); Record, Kerri, Boulder, CO Colorado 80303 (US)
(74) Representative: D Young & Co LLP

(56) References cited:
- GB-A- 1 462 033
- US-A- 3 830 676
- US-A- 5 989 285
- US-A- 6 113 626

## Description

### FIELD OF THE INVENTION

The present invention relates to medical pads, and more particularly, to medical pads utilized for thermal energy exchange with a patient. The invention is particularly apt pads utilized on the back region of a patient.

### BACKGROUND OF THE INVENTION

Increasingly, medical pads are being employed to achieve thermal exchange with patients. For example, medical pads have been widely employed to address emergency hypothermia or hyperthermia patient conditions. More recently, it has also been recognized that medical pads may be employed in conjunction with surgical procedures where selected thermal regulation of a patient is desired. For example, in procedures involving the exposure of bodily organs selective heating of the patient may be desirable.

To achieve thermal regulation in many medical pad systems, a heated or cooled fluid (e.g. air or water) is circulated through one or more pads which are contacted with a patient to affect surface-to-surface thermal energy exchange. As may be appreciated, the effectiveness of such thermal exchange is partially dependent upon the extent and intimacy of skin contact. In this regard, the establishment of the desired skin interface can be compromised where medical pads extend across bodily portions of differing complex configurations.

Additionally, the effectiveness of thermal exchange is partially dependant upon the maintenance of a desired thermal gradient across the medical pad-to-patient thermal exchange interface. In this regard, it should be noted that the maintenance of a predetermined rate of fluid circulation through the pad across the intended thermal exchange interface is important to achieving the desired thermal gradient.

Document US6113626 discloses a heat transfer blanket which wraps the torso and legs leaving the arms, buttocks, perineum and head exposed and allows for the selective heating or cooling of various body parts at the same or different rates.

### SUMMARY OF THE INVENTION

The present invention provides a pair of separate medical thermal exchange back pads as defined in claim 1.

Preferred features of the invention are defined in the dependent claims.

In a preferred embodiment, the provision of multiple channels of coincidental configurations facilitates the maintenance of a desired thermal gradient across the pad-to-patient interface, e.g. since any patient pressure occlusion within the fluid containing layer can be localized and fluid flow shunting can be minimized.

The provision of at least two different sets of fluid channels having corresponding coincidental configurations which are different enhances the ability to adapt the pad to conform to bodily portions of differing complex configurations, while also providing for a highly reliable and efficient degree of thermal exchange with a patient.

In a preferred embodiment, there is an intermediate fluid staging chamber for receiving fluid from one of the first and second plurality of fluid channels and distributing such fluid into the other of the first and second plurality of fluid channels. For such purposes, the first and second plurality of fluid channels may each have ends which terminate at the intermediate fluid staging chamber. Such an arrangement further facilitates the provision of a relatively even fluid flow through the various regions of the medical pad.

In a preferred embodiment, not being part of the invention, an end flap segment is pivotable about a pivot axis that is transverse to pivot axes of the side flap segments (e.g. at an angle of between about 70° to 110°). The first plurality of fluid channels is disposed so that each of the channels include a U-shaped portion located in one of the first and second side flap segments. Such segmentation and channeling features further facilitate the ability to achieve conformal positioning of the inventive pad on bodily portions having differing complex configurations.

In a preferred embodiment with at least one end flap segment pivotably interconnected to the central segment, the first and second ports of the fluid containing layer may be advantageously disposed within the end flap segment. Such positioning of the ports facilitates pad positioning on a patient, the ready establishment and maintenance of open fluid interconnections, and the localization of potential obstructions to medical personnel attending a patient. Further, the end flap segment may be pivoted relative to the central segment of the fluid containing layer so as to dispose the first and second ports away from bodily regions that may contact a patient support surface during medical procedures, thereby enhancing client comfort.

The first plurality of channels may be of a substantially common length, e.g. within about 15% of an average length thereof, as measured along their respective center paths. Similarly, each of the first plurality of channels may also have a substantially common average width, e.g. within about 25% of an average of their average widths. Similarly, the second plurality of channels may be of a substantially common length, e.g. within about 15% of an average length thereof, as measured along their respective center paths. The second plurality of channels may also have a substantially common average width, e.g. within about 25% of an average of their average widths. As may be appreciated, the provision of substantially common lengths within each channel set yields a substantially equal pressure drop from one end to the other of each of the channels comprising a given set. Further, the provision of substantially common widths further equalizes pressure drops and reduces flow shunting.

One of the channel sets may be provided within an average channel width that is significantly greater than an average channel width of the other set. For example, the average width of a first plurality of channels may be established to be at least 5 times greater, and preferably between about 10 to 25 times greater than an average channel width of a second plurality of channels.

In a preferred embodiment, not being part of the invention, the fluid ports are oriented to extend laterally away from the central segment. Additionally, such ports may be elongated and oriented to extend in substantially parallel co-relation. Such port orientation features can be implemented to reduce fluid line blockage considerations and facilitate patient access/comfort. Further, the ports may be provided with port members that are tapered to facilitate fluid flow and further enhance patient comfort.

In a preferred embodiment of the pair of back pads, because the central segments are adapted for positioning on the right and left sides of a patient's spine, the pads may be used on a wide range of patient sizes, with sequential positioning of the pads on a prone patient (e.g. by rolling the patient onto one shoulder then the other). The side flap segments are pivotably positioned relative to the central segments so as to accommodate positioning across the shoulder region, rib-cage region and/or back-to-hip/buttocks region of a patient. Additionally, a top end flap segment may be provided for pivotable positioning from the scapula to top shoulder region of a patient.

In one back pad embodiment, not being part of the invention, each of the back pads may be provided with fluid ports disposed in the corresponding end flap segments. More particularly, such ports may be of an elongated configuration and may be oriented to extend away from a patient, e.g. substantially parallel to the pivot axes corresponding with the end flap segments.

The adhesive surface extends across a majority and preferably all of the patient-facing side of the pad. The adhesive surface is defined by a conformable, thermally-conductive layer (e.g. a hydrogel layer). Further, adhesive strips with removable liners may be disposed along the outer side edges of the side flap segments and the top edge of the end flap segments of the pads. As may be appreciated, the liners on such adhesive strips may be selectively removed in conjunction with the liners provided on the above-noted conformable layer during positioning of the pads.

In addition to the noted features, the inventive pad may incorporate various teachings of U.S. Patent No. 6,197,045 entitled "COOLING/HEATING PAD AND SYSTEM", and U.S. Patent Application Serial No. 09/476,850 entitled "COOLING/HEATING PAD AND SYSTEM", filed January 3, 2000.

Additional aspects and advantages of the present invention will become apparent to those skilled in the art upon consideration of the further description provided hereinbelow.

### BRIEF DESCRIPTION OF THE DRAWINGS

Fig. 1 is a plan view of right and left back pads comprising one embodiment of the present invention.
Fig. 2 is an internal plan view of a fluid containing layer of the right back pad comprising the embodiment of Fig. 1.
Fig. 3 is a plan view of right and left back pads comprising another embodiment of the present invention.
Fig. 4 is an internal plan view of a fluid containing layer of the right back pad comprising the embodiment of Fig. 3.
Fig. 5 is a schematic illustration showing the right and left back pads of the embodiments of Figs. 1, 2 and Figs. 3, 4 fluidly interconnected with a fluid control system module.
Figs. 6A, 6B, 6C illustrate bodily front, side and back views, respectively, of the embodiment of Figs. 1 and 2 positioned on the back of a patient.
Fig. 7 is a perspective view of the embodiment of Figs. 1 and 2 as positioned on a prone patient.
Fig. 8 illustrates a portion of the fluid containing layer shown in Fig.2.
Fig. 9 is a top end view of the right back pad comprising the embodiment of Fig. 1.

### DETAILED DESCRIPTION

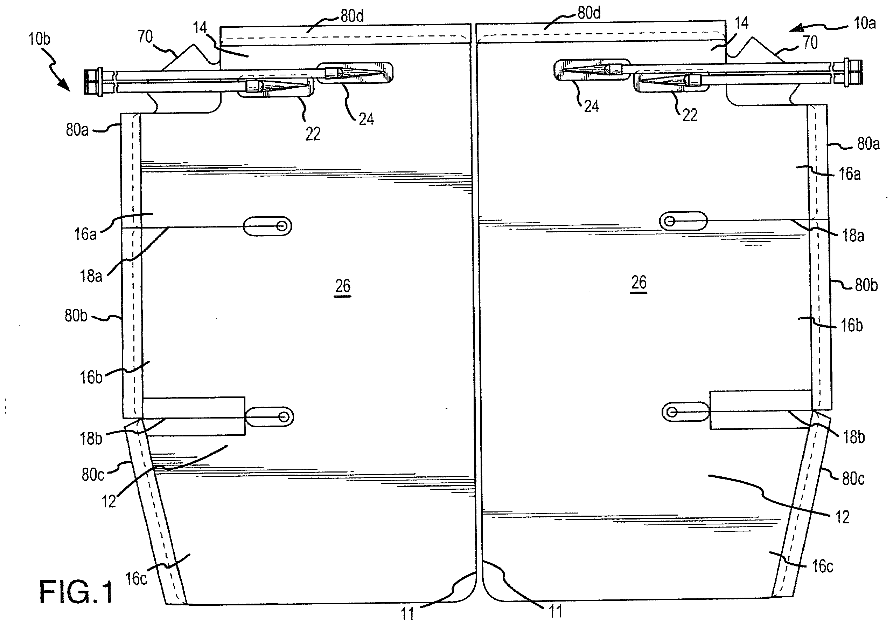

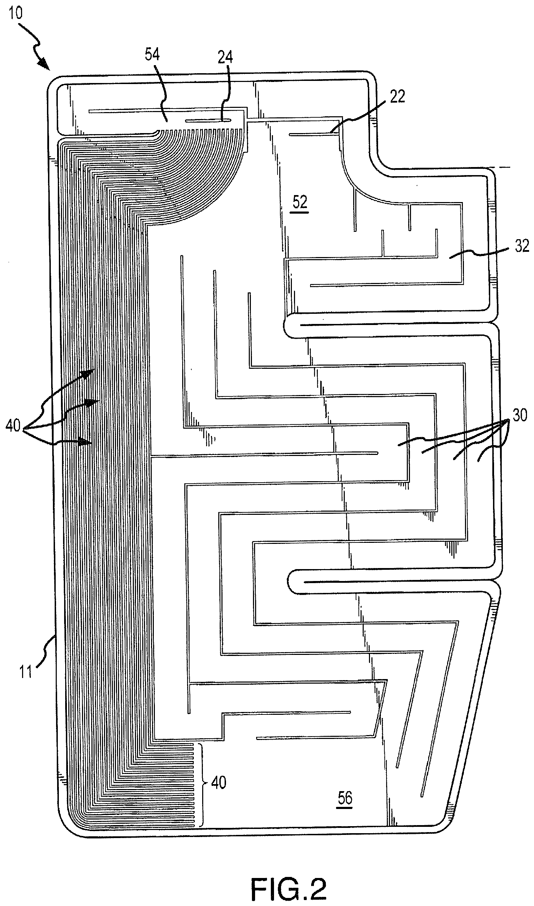

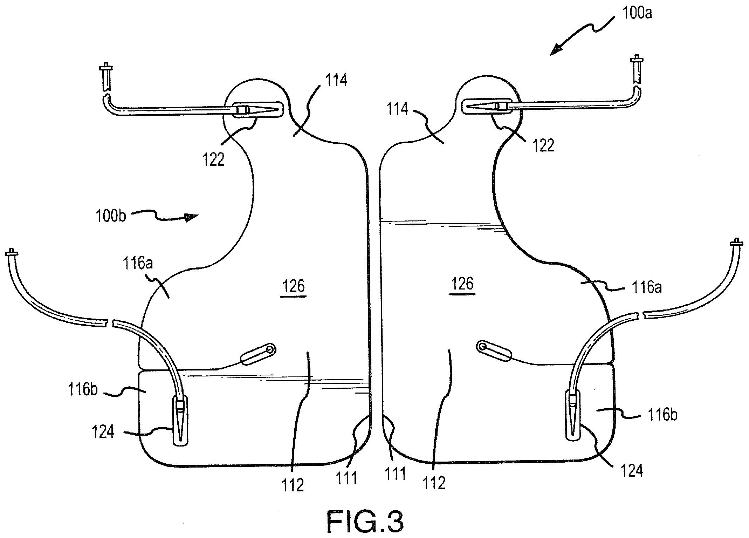

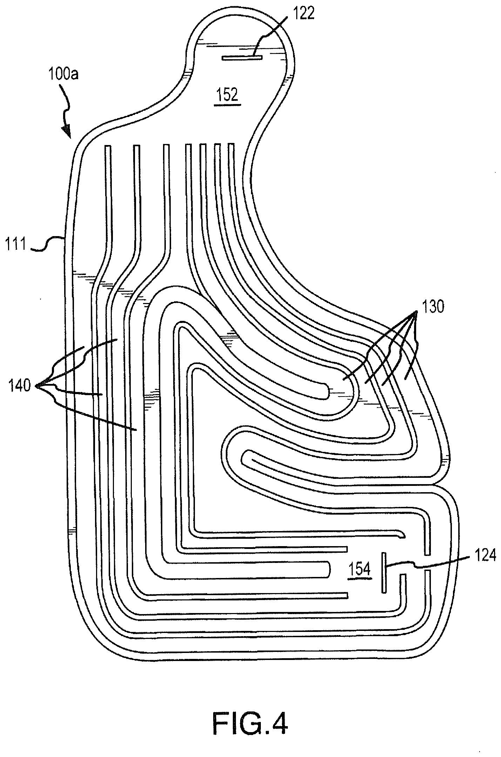

Figs. 1 and 2, and Figs. 3 and 4, correspond with two back pad embodiments 10 and 100, respectively, comprising features of the present invention. The two embodiments will be described in tandem, with differences therebetween identified when appropriate. Figs. 1 and 3 each show an external plan view of a pair of complimentary back pads 10a, 10b and 100a, 100b, respectively, intended for use on the right and left sides of a patient's back. Figs. 2 and 4 illustrate certain internal features of back pads 10a and 100a, respectively, wherein such features are the same in reverse orientation within back pads 10b and 100b, respectively.

Before proceeding it should be noted that various aspects of the invention are not limited to back pad applications. By way of example, channeling and segmentation features of the invention may be employed in conjunction with other anatomically configured pads, e.g. pads configured for use on one or both legs and/or the head of a patient.

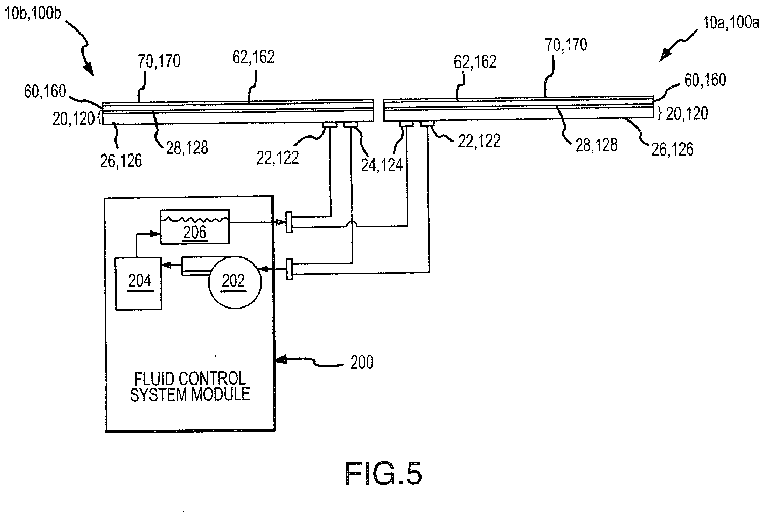

Referring now to the schematic illustration of Fig. 5, the illustrated back pad pairs 10a, 10b and 100a, 100b each include fluid containing layers 20, 120 through which fluid may be circulated during use. That is, fluid may be circulated through fluid ports 22, 24 and 122, 124 of the fluid containing layers 20 and 120, respectively, by an interconnectable fluid control system module 200 (e.g. via interconnected tubing lines). In one arrangement, module 200 includes a pump 202 for drawing fluid through the back pads 10a, 10b or 100a, 100b under negative pressure (e.g. less than about -10psi), at least one thermal exchange device 204 for heating and/or cooling the circulated fluid, and a fluid reservoir 206.

In addition to a fluid containing layer 20, 120, back pads 10, 100 each further include a conformable, thermally-conductive layer 60, 160 for contacting the skin of a patient. In this regard, the conformable layer 60, 160 defines an adhesive surface 62, 162 for enhancing the pad-to-skin interface. According to the invention, the adhesive surface 62, 162 extends across a major portion (e.g. substantially all) of the pads 10a, 10b. A release liner 70, 170 may also be provided on the adhesive surface 62, 162 for removal prior to use.

Each of the fluid containing layers 20, 120 may comprise opposing first and second layers 26, 28 and 126, 128, respectively, with a number of fluid channels defined by a plurality of rib members therewithin. More particularly, and as best shown by Figs. 2 and 4, fluid containing layers 20, 120 may include rib members that define a first plurality of adjacent fluid channels 30, 130 and a second plurality of adjacent fluid channels 40, 140 extending between the fluid ports 22, 24 and 122, 124 of the pads 10 and 100, respectively. As may be appreciated, fluid may be circulated from port 22, 122 to port 24, 124, or alternately from port 24, 124 to port 22, 124.

According to the invention, the first plurality of channels 30, 130 are of coincidental, serpentine configuration. More particularly, each of the channels comprising the first plurality of channels 30, 130 is of an S-shaped configuration. Further, such channels 30, 130 may be of a substantially common length, e.g. within about 15% of an average length as measured along their respective center paths. Channels 30, 130 may also have a substantially common average width, e.g. within about 25% of an average of their average widths.

The second plurality of channels 40, 140 are also disposed in a coincidental manner. As illustrated, a major portion of each of the channels comprising the second plurality of channels 40, 140 follows a substantially linear path. Further, it should be noted that channels 40, 140 may be of a substantially common length , e.g. within about 15% of an average length as measured along their respective center paths. Channels 40, 140 may also have a substantially common average width, e.g. within about 25% of an average of their average widths.

Fluid staging chambers 52, 152 and 54, 154 are provided at the fluid ports 22, 122 and 24, 154, respectively. Such staging chambers serve to distribute fluid and normalize fluid flow through the first plurality of channels 30, 130 and second plurality of channels 40, 140.

Additionally, in the back pad 10 of Fig. 1, an intermediate staging chamber 56 is provided between the first plurality of channels 30 and second plurality of channels 40. Such intermediate staging chamber 56 functions to distribute the flow of fluid from one of the first plurality of channels 30 and second plurality of channels 40 to the other, depending on the direction of fluid flow. In this regard, it should be noted that in the back pad 10, a closed fluid flow path between ports 22 and 24 is defined through the first plurality of channels 30, intermediate staging chamber 56 and the second plurality of channels 40.

From an overall configuration standpoint, and with particular reference to Figs. 1 and 3, back pads 10, 100 are of mirrored configuration, and each include a central segment 12, 112 having a number of flap segments pivotably adjoined thereto. The provision of multiple flap segments facilitates the conformal positioning of back pads 10, 100 about the sides and shoulders of a patient.

In the later regard, back pads 10, 100 each include an end flap segment 14, 114 for conformal positioning between the scapula and top shoulder region of a patient. Further, back pads 10, 100 each include a plurality of side flap segments 16a, 16b, 16c and 116a, 116b, respectively, separated by slits for pivotable, conformable positioning about the sides of a patient. Specifically, the side flap segments 16a, 16b, 16c of back pad 10 are physically separated by slits 18a, 18b, thereby allowing for the separate manipulation of each of the side flap segments 16a, 16b, 16c. For example, side flap segment 16a can be positioned across the upper back to outer shoulder region on a patient, side flap segment 16b may be positioned about the ribcage of a patient, and side flap segment 16c may be positioned about the lower back-to-hip/buttocks region of a patient. Further, it should be noted that the side flap segments 16a, 16b, 16c may be separately detached from or left unattached to a patient during a medical procedure. In particular, the middle flap segment 16b may be manipulated to allow for access to the abdominal region through the ribcage (e.g. for purposes of placing chest drainage tubes or monitoring electrodes).

In order to accommodate the end flap and side flap segments noted above, while also providing for effective thermal exchange with a patient, the configurations and relative widths of the first plurality of channels 30, 130 and second plurality of channels 40, 140, should be further addressed. According to the invention, and as best shown in Figs. 3 and 5, the first plurality of channels 30, 130 each include a U-shaped portion extending through the side flap segments 16b, 116a. In pads 10, 100, the pivot axes of the various side flap segments are substantially parallel to the basis of the U-shaped portions of channels 30, 130, as well as the outer side edges of the side flap segments. Further, and with particular respect to back pad 10, it can be seen that an additional channel 32 is configured in a serpentine fashion to pass through side flap segment 16a as well as end flap segment 14.

Referring further to Figs. 3 and 5, it should be noted that the above-noted linear path portions of the second plurality of channels 40, 140 extend substantially parallel to a side edge 11, 111 of the back pad 10, 100. As may be appreciated, such side edge 11, 111 is intended for positioning substantially parallel to the spine of a patient.

In that regard, in back pad 10 the second plurality of channels 40 have an average channel width that is less than the average channel width of the first plurality of channels 30. More particularly, the average channel width of in the second plurality of channels 40 is at least about 5 times, and more preferably about 10 to 25 times less than the average channel width for the first plurality of channels 30. Such condensed spacing of the channels within the second plurality of channels 40 facilitates support of the second layer 28 over the first layer 26 of the fluid containing layer 20.

Relatedly, it should also be noted that for such support purposes, back pads 10, 100 may include a matrix of support dimples extending between the first and second layers 26, 28 and 126, 128 of the fluid containing layers 20 and 120, respectively. By way of example, such matrix may be defined by offsetting rows and columns of frusto-conical dimples which extend from the first layer 26 to the second layer 28.

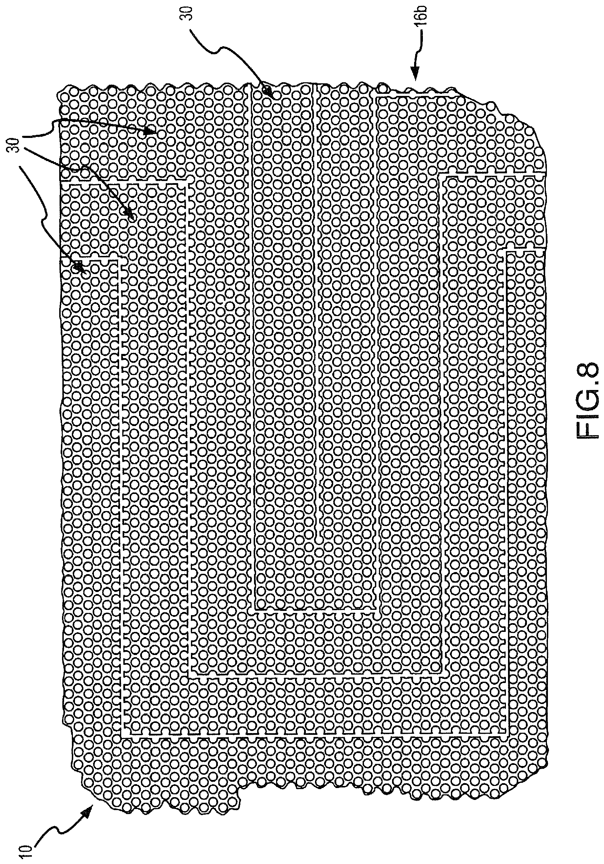

In back pads 10, 100 the dimple matrix may be provided across the first plurality of channels 30, 130, as well as the fluid staging chambers 52, 152 and 54, 154. Additionally, the dimple matrix may be provided across the intermediate staging chamber 56 and channel 32 of back pad 10. Further, the dimple matrix may also extend across the second plurality of channels 140 in pad 100. Fig. 8 shows an exemplary dimple matrix within a portion of the side flap segment 16b of pad 10.

In one fabrication approach, the first layers 26, 126 of the back pad 10, 100 may comprise a molded foam material that integrally defines the noted dimple matrices as well as the rib members which define the various fluid channels comprising the fluid containing layers 20, 120. Additionally, the molded foam material may define the noted dimple matrices. The second layers 28, 128 of the fluid containing layers 20, 120 may comprise a flexible membrane (e.g. polyolefin, polyurethane, flexible polyvinylchloride) that is sealed around the periphery and across interfacing top end portions of the various ribs and dimples comprising the fluid containing layers 20, 120.

Further, the conformable layer 60, 160 may comprise a first material suspended in a matrix defined by a second material. By way of example, the first material may comprise a liquid and the second material may comprise a polymer. More particularly, conformable layer 60, 160 may comprise a hydrogel that yields an adhesive surface 62, 162 that provides a desirable adhesive and conformable interface with the skin of a patient. As may be appreciated, the utilization of such hydrogel material also facilitates thermal exchange between the fluid containing layer 20, 120 of back pad 10, 100 and a patient.

In relation to the foregoing, it should be noted that the first layers 26, 126, second layers 28, 128, conformable layers 60, 160 and dimple matrices may be provided utilizing the teachings of U.S. Patent No. 6,197,045 entitled "COOLING/HEATING PAD AND SYSTEM", and U.S. Patent Application Serial No. 09/476,850 entitled "COOLING/HEATING PAD AND SYSTEM", filed January 3, 2000.

In addition to the above-noted features of back pad 10, 100, a number of optional features may be included. In particular, and with particular reference to Fig. 1, back pad 10 may comprise a number of peripheral adhesive strips 80 each having a selectively removable release liner exposed thereupon. For example, strips 80 may comprise a polyolefin or polyurethane film with hypoallergenic pressure sensitive acrylate adhesive anchored to the pad 10, 100 with a rubber based pressure sensitive adhesive.

As illustrated, adhesive strips 80a, 80b, 80c are located on the side segments 16a, 16b, 16c, respectively, for selective removal upon positioning the back pad 10 on a patient. That is, such adhesive strips 80a, 80b, 80c may be utilized to facilitate the securement of the edges of side flap segment 16a, 16b, 16c to a patient. Similarly, an adhesive strip 80d may be provided along the end flap segment 14 for selective use in securing the end flap segment 14 to a patient.

Further, the location, configuration and orientation of ports 22, 122 and 24, 124 may be selectively established to provide various advantages. In particular, ports 22, 122 and 24, 124 may be provided to avoid patient weight from creating localized high pressure areas on the skin by pressing the port or attached tubing against the skin of a patient. Reducing such high pressure areas reduces any risk of pressure ulcers. Also, the tubing can exit off an operating table without multiple turns, thereby reducing any risk of interconnected tubing buckling/kinking which limits fluid flow.

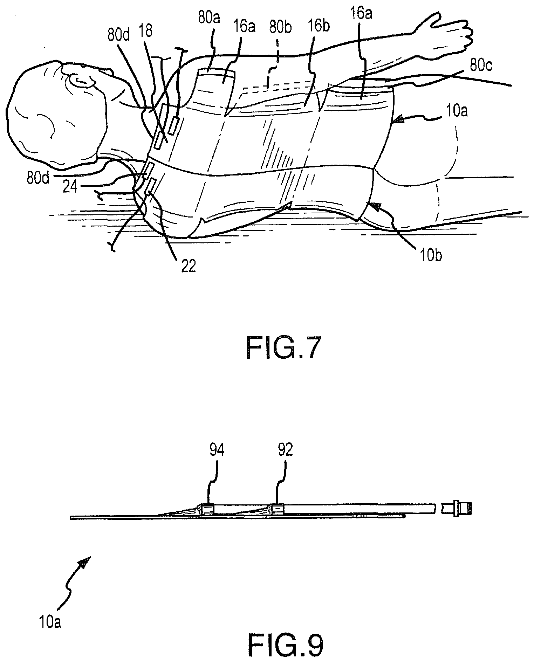

Referring in particular to Figs. 1 and 9, the illustrated ports 22, 24 will now be described in further detail. As shown, ports 22 and 24 are both provided in the end flap segment 14. Further, it can be seen that ports 22 and 24 each include an elongated opening through the first layer 26 into the fluid containing layer 20. Correspondingly, elongated port members 92, 94 are interconnected to the outside, exposed surface of the first layer 26, wherein the elongated openings of ports 22, 24 are in aligned relation with the elongated port members 92, 94. Further in that regard, it can be seen that the center axes for ports 22, 24 and the corresponding port members 92, 94 extend laterally away from the peripheral edge 11 and central segment 12 of the back pad 10 in parallel co-relation. As best shown by Fig. 9, the port members 92, 94 are also tapered as they extend laterally outward to accommodate patient comfort considerations and provide a smooth fluid flow transition.

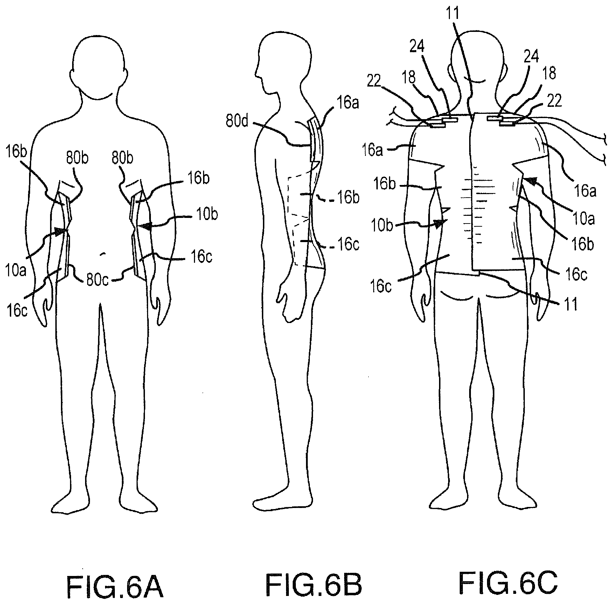

To further facilitate an appreciation of various aspects of the present invention, an exemplary use of back pads 10a, 10b will now be further described with reference to Figs. 6A, 6B, 6C and 7. Initially, it should be noted that back pads 10a, 10b may be provided in a plurality of different sized sets. As such, use of back pad 10a, 10b may first entail the selection of the best fit set, e.g. based on patient height.

While pads 10a, 10b may be positioned in either order, Figs. 6A, 6B, 6C illustrate initial positioning of pad 10b. For such positioning, the release liner 70 may be removed from pad 10b and the middle flap segment 16b may be positioned along the mid-axilla of a patient. Then, the bony prominence at the top of the spine of the patient (i.e. protruding just below the neck region) may be located. If the patient is sitting upright, the top edge of the end flap segment 14 of the back pad 10b may be located along a lateral line that extends cross-wise a few inches below the noted bony prominence. If the patient is lying down, such top edge may be placed closer to the lateral line. Of note, if the patient is lying down, the patient may be rolled onto his/her right shoulder during placement of pad 10b.

Next, the upper side flap segment 16a may be lightly positioned on the triceps area of a patient. Further, the pad 10b may be lightly pressed across its lateral extent. At this point, the liners may be removed from the adhesive strips 80b, 80c, 80d, whereupon the adhesive strips are secured to the interfacing skin regions of a patient. Thereafter, the arms of a patient can be tucked to the patient's side and the top side flap segment 16a repositioned thereupon. To maintain such position of segment 16a, the liner may be removed from the adhesive strip 80a and such strip 80a may be secured to the patient's skin.

Following placement of back pad 10b, back pad 10a may be positioned utilizing a similar procedure. If the patient is lying down, the patient may be rolled onto his/her left shoulder before placement of pad 10a. As may be appreciated, back pad 10a may be conveniently overlapped on back pad 10b as necessary along the spinal interface region. Such overlap may be advantageously maintained due to the adhesive surface 62 presented by the conformable layer 60. Such overlap capability facilitates utilization of pads 10a, 10b on a variety of patient sizes.

As shown in Fig. 7, various pivot axes are provided in relation to the side flaps segments 16a, 16b, 16c and end flap segment 18. In this regard, it may be noted that the pivot axis for end flap segment 18 preferably forms an angle of between about 70° and 110° relative to the pivot axes of side flap segments 16a, 16b, 16c. Such relative orientation of the axes accommodates conformable positioning of the pads 10a, 10b across the various corresponding body regions (e.g. shoulders, rib cage, lower back-to-hip/buttocks regions) which have differing, complex configurations.

As also shown in Fig. 7, positioning of ports 22, 24 in end flap segment 14 allows for ready access thereto and avoids patient discomfort that could result from "sandwiching" of such ports between a patient and a support surface. Further, such positioning directs fluid tubing that is interconnected to ports 22, 24 laterally away from the patient, thereby reducing kinking considerations and otherwise reducing potential obstructions for patient care by medical personnel.

## Claims

1. A pair of separate medical thermal exchange back pads, comprising:
a first back pad (10a, 100a), including:
a side edge (11, 111) for positioning on a patient's back parallel to a patient's spine;
a pliable fluid containing layer (20, 120) for containing a fluid circulated from a first port (22, 122) to a second port (24, 124) of the fluid containing layer, wherein as part of the first back pad the fluid containing layer (20, 120) includes a central segment (12, 112) for positioning on a right side of a patient's spine, and first and second side flap segments (16a-16c; 116a-116b) separately and pivotably interconnected to the central segment for separate manipulation and positioning across a patient's rib-cage region on said right side of a patient's spine with said side edge of the first back pad positioned on a patient's back parallel to the patient's spine;
a first plurality of serpentine fluid channels (30, 130) defined within said fluid containing layer, wherein said plurality of fluid channels each include at least a U-shaped first portion located in one of said first and second side flap segments, respectively;
a second plurality of channels (40, 140) defined within said fluid containing layer and fluidly interconnected with the first plurality of serpentine channels (30, 130), wherein a major portion of each of the channels comprising the second plurality of channels (40, 140) follows a substantially linear path parallel to said side edge (11, 111) with said side edge of the first back pad positioned on a patient's back parallel to the patient's spine;
a conformable layer disposed on a first side of the fluid containing layer (20, 120), said conformable layer being thermally-conductive and defining an adhesive surface (62, 162) extending across a majority of a patient-facing side of the first back pad for contacting skin of a patient; and,
a second back pad (10b, 110b), separate from the first back pad (10a, 110a), including:
a side edge (11, 111) for positioning on a patient's back parallel to a patient's spine;
a pliable fluid containing layer (20, 120) for containing a fluid circulated from a first port (22, 122) to a second port (24, 124) of the fluid containing layer, wherein as part of the second back pad the fluid containing layer (20, 120) includes a central segment (12, 112) for positioning on a left side of a patient's spine, and first and second side flap segments (16a-16c; 116a-116b) separately and pivotably interconnected to the central segment for separate manipulation and positioning across a patient's rib-cage region on said left side of a patient's spine with said side edge of the second back pad positioned on a patient's back parallel to the patient's spine;
a first plurality of serpentine fluid channels (30, 130) defined within said fluid containing layer, wherein said plurality of fluid channels each include at least a U-shaped first portion located in one of said first and second side flap segments, respectively;
a second plurality of channels (40, 140) defined within said fluid containing layer and fluidly interconnected with the first plurality of serpentine channels (30, 130), wherein a major portion of each of the channels comprising the second plurality of channels (40, 140) follows a substantially linear path parallel to said side edge (11, 111) of the second back pad;
a conformable layer disposed on a first side of the fluid containing layer (20, 120), said conformable layer being thermally-conductive and defining an adhesive surface (62, 162) extending across a majority of a patient-facing side of the second back pad for contacting skin of a patient.

2. A pair of separate medical thermal exchange back pads as recited in Claim 1, wherein for each of said first back pad and said second back pad said first plurality of fluid channels each pass through said first and second side flap segments to define an S-shaped configuration.

3. A pair of separate medical thermal exchange back pads as recited in Claim 1, wherein for each of said first back pad and said second back pad said pivot axes corresponding with said first and second side flap segments are substantially parallel to said side edge (11, 111).

4. A pair of separate medical thermal exchange back pads as recited in Claim 1, each of said first back pad and said second back pad further comprising:
a release liner (70, 170) provided on the adhesive surface and removable prior to use.

5. A pair of separate medical thermal exchange back pads as recited in Claim 1, each of said first back pad and second back pad further comprising:
an inlet fluid staging chamber (52, 152) defined within said fluid containing layer, for distributing fluid from said first port into said first plurality of fluid channels, wherein said first plurality of fluid channels each have an inlet end that terminates at said inlet fluid staging chamber.

6. A pair of separate medical thermal exchange back pads as recited in Claim 1, wherein for each of said first back pad and said second back pad said fluid containing layer is defined by opposing top and bottom layers (26, 28; 126, 128), and said plurality of first fluid channels being defined by a plurality of rib members adjoined to and between said opposing top and bottom layers.

7. A pair of separate medical thermal exchange back pads as recited in Claim 6, wherein for each of said first back pad and said second back pad said plurality of rib members are integrally defined by said bottom layer.

8. A pair of separate medical thermal exchange back pads as recited in Claim 7, wherein for each of said first back pad and said second back pad said top layer is defined by a flexible film.

9. A pair of separate medical thermal exchange back pads as recited in Claim 8, wherein for each of said first back pad and said second back pad said bottom layer further integrally defines a matrix of upstanding dimples, and wherein said matrix of dimples extends across said plurality of fluid channels.

10. A pair of separate medical thermal exchange back pads as recited in Claim 1, wherein for each of said first back pad and said second back pad said conformable layer comprises:
a first material suspended in a matrix defined by a second material.

11. A pair of separate medical thermal exchange back pads as recited in Claim 10, wherein for each of said first back pad and said second back pad said first material comprises a liquid and said second material comprises a polymer.

12. A pair of separate medical thermal exchange back pads as recited in Claim 1, wherein for each of said first back pad and said second back pad said conformable layer comprises a hydrogel material.

13. A pair of separate medical thermal exchange back pads as recited in Claim 1, wherein for each of said first back pad and said second back pad, one of the first port and the second port is located in one of the first and second side flap segments.

14. An arrangement comprising a pair of separate medical thermal exchange back pads according to any of Claims 1-13, and further comprising:
a pump,
wherein the first port and the second port of the first back pad are configured to be interconnected to the pump via interconnected tubing lines for circulation of a fluid by the pump through the first and second plurality of channels of the fluid containing layer of the first back pad; and,
wherein the first port and the second port of the second back pad are configured to be interconnected to the pump via interconnected tubing lines for circulation of a fluid by the pump through the first and second plurality of channels of the fluid containing layer of the first back pad.

15. An arrangement as recited in Claim 14, wherein said pump is configured to draw said fluid through said first back pad under negative pressure, and wherein said pump is configured to draw said fluid through said second back pad under negative pressure.

## Patentansprüche

1. Paar von separaten medizinischen Wärmetauschauflagen für den Rücken, umfassend:
eine erste Rückenauflage (10a, 100a), einschließend:
einen Seitenrand (11, 111) zum Positionieren auf dem Rücken eines Patienten parallel zu der Wirbelsäule eines Patienten;
eine nachgiebige fluidhaltige Schicht (20, 120) zum Enthalten eines Fluids, das von einem ersten Anschluss (22, 122) zu einem zweiten Anschluss (24, 124) der fluidhaltigen Schicht zirkuliert wird, wobei die fluidhaltige Schicht (20, 120) als Teil der ersten Rückenauflage ein zentrales Segment (12, 112) zum Positionieren auf der rechten Seite der Wirbelsäule eines Patienten sowie erste und zweite Seitenklappensegmente (16a-16c; 116a-116b) einschließt, die separat und schwenkbar mit dem zentralen Segment verbunden sind, um separat manipuliert und über einer Brustkorbregion eines Patienten auf der rechten Seite der Wirbelsäule eines Patienten positioniert zu werden, wobei der Seitenrand der ersten Rückenauflage auf dem Rücken eines Patienten parallel zu der Wirbelsäule des Patienten positioniert wird;
eine erste Vielzahl von serpentinenartigen Fluidkanälen (30, 130), die in der fluidhaltigen Schicht definiert sind, wobei die Vielzahl der Fluidkanäle jeweils mindestens einen U-förmigen ersten Anteil einschließt, der sich in einem von dem ersten beziehungsweise zweiten Seitenklappensegment befindet;
eine zweite Vielzahl von Kanälen (40, 140), die in der fluidhaltigen Schicht definiert sind und fluidtechnisch mit der ersten Vielzahl von serpentinenartigen Kanälen (30, 130) verbunden sind, wobei ein größerer Anteil von jedem der Kanäle, welche die zweite Vielzahl der Kanäle (40, 140) umfassen, einem im Wesentlichen linearen Pfad parallel zu dem Seitenrand (11, 111) folgt, wobei der Seitenrand der ersten Rückenauflage auf dem Rücken eines Patienten parallel zu der Wirbelsäule des Patienten positioniert ist;
eine anschmiegsame Schicht, die auf einer ersten Seite der fluidhaltigen Schicht (20, 120) angeordnet ist, wobei die anschmiegsame Schicht wärmeleitfähig ist und eine Haftoberfläche (62, 162) definiert, die sich über einen Großteil einer zum Patienten weisenden Seite der ersten Rückenauflage erstreckt, um in Kontakt mit der Haut eines Patienten zu kommen; und
eine zweite Rückenauflage (10b, 110b), die separat von der ersten Rückenauflage (10a, 110a) ist, einschließend:
einen Seitenrand (11, 111) zum Positionieren auf dem Rücken eines Patienten parallel zu der Wirbelsäule eines Patienten;
eine nachgiebige fluidhaltige Schicht (20, 120) zum Enthalten eines Fluids, das von einem ersten Anschluss (22, 122) zu einem zweiten Anschluss (24, 124) der fluidhaltigen Schicht zirkuliert wird, wobei die fluidhaltige Schicht (20, 120) als Teil der zweiten Rückenauflage ein zentrales Segment (12, 112) zum Positionieren auf einer linken Seite der Wirbelsäule eines Patienten sowie erste und zweite Seitenklappensegmente (16a-16c; 116a-116b) einschließt, die separat und schwenkbar mit dem zentralen Segment verbunden sind, um separat manipuliert und über einer Brustkorbregion eines Patienten auf der linken Seite der Wirbelsäule eines Patienten positioniert zu werden, wobei der Seitenrand der zweiten Rückenauflage auf dem Rücken eines Patienten parallel zu der Wirbelsäule des Patienten positioniert wird;
eine erste Vielzahl von serpentinenartigen Fluidkanälen (30, 130), die in der fluidhaltigen Schicht definiert sind, wobei die Vielzahl der Fluidkanäle jeweils mindestens einen U-förmigen ersten Anteil einschließt, der sich in einem von dem ersten beziehungsweise zweiten Seitenklappensegment befindet;
eine zweite Vielzahl von Kanälen (40, 140), die in der fluidhaltigen Schicht definiert sind und fluidtechnisch mit der ersten Vielzahl von serpentinenartigen Kanälen (30, 130) verbunden sind, wobei ein größerer Anteil von jedem der Kanäle, welche die zweite Vielzahl der Kanäle (40, 140) umfassen, einem im Wesentlichen linearen Pfad parallel zu dem Seitenrand (11, 111) der zweiten Rückenauflage folgt;
eine anschmiegsame Schicht, die auf einer ersten Seite der fluidhaltigen Schicht (20, 120) angeordnet ist, wobei die anschmiegsame Schicht wärmeleitfähig ist und eine Haftoberfläche (62, 162) definiert, die sich über einen Großteil einer zum Patienten weisenden Seite der zweiten Rückenauflage erstreckt, um in Kontakt mit der Haut eines Patienten zu kommen.

2. Paar von separaten medizinischen Wärmetauschauflagen für den Rücken nach Anspruch 1, wobei für jede von der ersten Rückenauflage und der zweiten Rückenauflage die erste Vielzahl von Fluidkanälen jeweils das erste und das zweite Seitenklappensegment passiert, um eine S-förmige Konfiguration zu definieren.

3. Paar von separaten medizinischen Wärmetauschauflagen für den Rücken nach Anspruch 1, wobei für jede von der ersten Rückenauflage und der zweiten Rückenauflage die Schwenkachsen, die dem ersten und dem zweiten Klappensegment entsprechen, im Wesentlichen parallel zu dem Seitenrand (11, 111) sind.

4. Paar von separaten medizinischen Wärmetauschauflagen für den Rücken nach Anspruch 1, wobei jede von der ersten Rückenauflage und der zweiten Rückenauflage des Weiteren umfasst:
eine Trennfolie (70, 170), die auf der Haftoberfläche bereitgestellt wird und vor Gebrauch zu entfernen ist.

5. Paar von separaten medizinischen Wärmetauschauflagen für den Rücken nach Anspruch 1, wobei jede von der ersten Rückenauflage und der zweiten Rückenauflage des Weiteren umfasst:
eine Einlassfluidstufenkammer (52, 152), die in der fluidhaltigen Schicht definiert ist, um Fluid von dem ersten Anschluss in die erste Vielzahl der Fluidkanäle zu verteilen, wobei die erste Vielzahl der Fluidkanäle jeweils ein Einlassende aufweist, welches in der Einlassfluidstufenkammer endet.

6. Paar von separaten medizinischen Wärmetauschauflagen für den Rücken nach Anspruch 1, wobei für jede von der ersten Rückenauflage und der zweiten Rückenauflage die fluidhaltige Schicht durch sich gegenüberliegende Oberschichten und Unterschichten (26, 28; 126, 128) definiert ist, und die Vielzahl der ersten Fluidkanäle durch eine Vielzahl von Rippenelementen definiert ist, die an die sich gegenüberliegenden Ober- und Unterschichten angrenzen und zwischen diesen liegen.

7. Paar von separaten medizinischen Wärmetauschauflagen für den Rücken nach Anspruch 6, wobei für jede von der ersten Rückenauflage und der zweiten Rückenauflage die Vielzahl der Rippenelemente integral durch die Unterschicht definiert ist.

8. Paar von separaten medizinischen Wärmetauschauflagen für den Rücken nach Anspruch 7, wobei die Oberschicht für jede von der ersten Rückenauflage und der zweiten Rückenauflage durch einen flexiblen Film definiert ist.

9. Paar von separaten medizinischen Wärmetauschauflagen für den Rücken nach Anspruch 8, wobei die Unterschicht für jede von der ersten Rückenauflage und der zweiten Rückenauflage des Weiteren integral eine Matrix von aufrecht stehenden Grübchen definiert, und wobei die Matrix der Grübchen sich über die Vielzahl der Fluidkanäle erstreckt.

10. Paar von separaten medizinischen Wärmetauschauflagen für den Rücken nach Anspruch 1, wobei die anschmiegsame Schicht für jede von der ersten Rückenauflage und der zweiten Rückenlage umfasst:
ein erstes Material, das in einer Matrix suspendiert ist, die durch ein zweites Material definiert ist.

11. Paar von separaten medizinischen Wärmetauschauflagen für den Rücken nach Anspruch 10, wobei für jede von der ersten Rückenauflage und der zweiten Rückenauflage das erste Material eine Flüssigkeit umfasst und das zweite Material ein Polymer umfasst.

12. Paar von separaten medizinischen Wärmetauschauflagen für den Rücken nach Anspruch 1, wobei die anschmiegsame Schicht für jede von der ersten Rückenauflage und der zweiten Rückenlage ein Hydrogelmaterial umfasst.

13. Paar von separaten medizinischen Wärmetauschauflagen für den Rücken nach Anspruch 1, wobei für jede von der ersten Rückenauflage und der zweiten Rückenauflage einer von dem ersten Anschluss und dem zweiten Anschluss sich in einem von dem ersten und dem zweiten Seitenklappensegment befindet.

14. Anordnung, umfassend ein Paar von separaten medizinischen Wärmetauschauflagen für den Rücken nach einem der Ansprüche 1 bis 13, und des Weiteren umfassend:
eine Pumpe,
wobei der erste Anschluss und der zweite Anschluss der ersten Rückenauflage ausgestaltet sind, um über verbundene Schlauchleitungen zur Zirkulation eines Fluids mittels der Pumpe durch die erste und zweite Vielzahl der Kanäle der fluidhaltigen Schicht der ersten Rückenauflage mit der Pumpe verbunden zu werden; und
wobei der erste Anschluss und der zweite Anschluss der zweiten Rückenauflage ausgestaltet sind, um über verbundene Schlauchleitungen zur Zirkulation eines Fluids mittels der Pumpe durch die erste und zweite Vielzahl der Kanäle der fluidhaltigen Schicht der ersten Rückenauflage mit der Pumpe verbunden zu werden.

15. Anordnung nach Anspruch 14, wobei die Pumpe ausgestaltet ist, um das Fluid unter Negativdruck durch die erste Rückenauflage zu ziehen, und wobei die Pumpe ausgestaltet ist, um das Fluid unter Negativdruck durch die zweite Rückenauflage zu ziehen.

## Revendications

1. Paire de garnitures dorsales à échange thermique médicales séparées, comprenant :
une première garniture dorsale (10a, 100a), comportant :
un bord latéral (11, 111) pour un positionnement sur le dos d'un patient parallèlement à la colonne vertébrale du patient ;
une couche pliable (20, 120) contenant un fluide pour contenir un fluide mis à circuler depuis un premier orifice (22, 122) vers un second orifice (24, 124) de la couche contenant un fluide, dans lequel, en tant que partie de la première garniture dorsale, la couche (20, 120) contenant un fluide comprend un segment central (12, 112) pour un positionnement sur un côté droit de la colonne vertébrale du patient, et des premier et second segments (16a à 16c ; 116a à 116b) de rabat latéral interconnectés de manière séparée et pivotante au segment central pour une manipulation et un positionnement séparés en travers de la région de la cage thoracique du patient sur ledit côté droit de la colonne vertébrale du patient, ledit bord latéral de la première garniture dorsale étant positionné sur le dos du patient parallèlement à la colonne vertébrale du patient ;
une première pluralité de canaux de fluide en serpentin (30, 130) définie à l'intérieur de ladite couche contenant un fluide, dans laquelle chaque canal de ladite pluralité de canaux de fluide comprend au moins une première partie en U située dans un desdits premier et second segments de rabat latéral, respectivement ;
une seconde pluralité de canaux (40, 140) définie à l'intérieur de ladite couche contenant un fluide et interconnectés fluidiquement avec la première pluralité de canaux en serpentin (30, 130), dans laquelle une grande partie de chacun des canaux comprenant la seconde pluralité de canaux (40, 140) suit un trajet sensiblement linéaire parallèle audit bord latéral (11, 111), ledit bord latéral de la première garniture dorsale étant positionné sur le dos du patient parallèlement à la colonne vertébrale du patient ;
une couche conforme disposée sur un premier côté de la couche (20, 120) contenant un fluide, ladite couche conforme étant thermoconductrice et définissant une surface adhésive (62, 162) s'étendant en travers d'une majeure partie d'un côté tourné vers le patient de la première garniture dorsale pour entrer en contact avec la peau du patient ; et,
une seconde garniture dorsale (10b, 110b), distincte de la première garniture dorsale (10a, 110a), comportant :
un bord latéral (11, 111) pour un positionnement sur le dos du patient parallèlement à la colonne vertébrale du patient ;
une couche pliable (20, 120) contenant un fluide pour contenir un fluide mis à circuler depuis un premier orifice (22, 122) vers un second orifice (24, 124) de la couche contenant un fluide, dans laquelle en tant que partie de la seconde garniture dorsale, la couche (20, 120) contenant un fluide comprend un segment central (12, 112) pour un positionnement sur un côté gauche de la colonne vertébrale du patient, et les premier et second segments (16a à 16c ; 116a à 116b) de rabat latéral étant interconnectés de manière séparée et pivotante au segment central pour une manipulation et un positionnement séparés en travers de la région de la cage thoracique du patient sur ledit côté gauche de la colonne vertébrale du patient, ledit bord latéral de la seconde garniture dorsale étant positionné sur le dos du patient parallèlement à la colonne vertébrale du patient ;
une première pluralité de canaux de fluide en serpentin (30, 130) définis à l'intérieur de ladite couche contenant un fluide, chaque canal de ladite pluralité de canaux de fluide comprenant au moins une première partie en U située dans un desdits premier et second segments de rabat latéral, respectivement ;
une seconde pluralité de canaux (40, 140) définis à l'intérieur de ladite couche contenant un fluide et interconnectés fluidiquement avec la première pluralité de canaux en serpentin (30, 130), dans laquelle une grande partie de chacun des canaux comprenant la seconde pluralité de canaux (40, 140) suit un trajet sensiblement linéaire parallèle audit bord latéral (11, 111) de la seconde garniture dorsale ;
une couche conforme disposée sur un premier côté de la couche (20, 120) contenant un fluide, ladite couche conforme étant thermoconductrice et définissant une surface adhésive (62, 162) s'étendant en travers d'une majeure partie d'un côté tourné vers le patient de la seconde garniture dorsale pour entrer en contact avec la peau du patient.

2. Paire de garnitures dorsales à échange thermique médicales séparées selon la revendication 1, dans laquelle pour chacune de ladite première garniture dorsale et de ladite seconde garniture dorsale, lesdits multiples canaux de fluide passent chacun à travers lesdits premier et second segments de rabat latéral pour définir une configuration en S.

3. Paire de garnitures dorsales à échange thermique médicales séparées selon la revendication 1, dans laquelle pour chacune de ladite première garniture dorsale et de ladite seconde garniture dorsale, lesdits axes de pivot correspondant auxdits premier et second segments de rabat latéral sont sensiblement parallèles audit bord latéral (11, 111).

4. Paire de garnitures dorsales à échange thermique médicales séparées selon la revendication 1, chacune de ladite première garniture dorsale et de ladite seconde garniture dorsale comprenant en outre :
une doublure antiadhésive (70, 170) prévue sur la surface adhésive et amovible avant utilisation.

5. Paire de garnitures dorsales à échange thermique médicales séparées selon la revendication 1, chacune desdites première garniture dorsale et seconde garniture dorsale comprenant en outre :
une chambre d'étagement de fluide d'admission (52, 152) définie à l'intérieur de ladite couche contenant un fluide, pour distribuer un fluide depuis ledit premier orifice vers ladite première pluralité de canaux de fluide, dans lequel les canaux de ladite première pluralité de canaux de fluide possèdent chacun une extrémité d'admission qui se termine au niveau de ladite chambre d'étagement de fluide d'admission.

6. Paire de garnitures dorsales à échange thermique médicales séparées selon la revendication 1, dans laquelle pour chacune de ladite première garniture dorsale et de ladite seconde garniture dorsale, dans laquelle ladite couche contenant un fluide est définie par des couches supérieure et inférieure opposées (26, 28 ; 126, 128), et ladite pluralité de premiers canaux de fluide est définie par une pluralité d'éléments de nervures contigus auxdites couches supérieure et inférieure opposées et entre celles-ci.

7. Paire de garnitures dorsales à échange thermique médicales séparées selon la revendication 6, dans laquelle pour chacune de ladite première garniture dorsale et de ladite seconde garniture dorsale, ladite pluralité d'éléments de nervure est entièrement définie par ladite couche inférieure.

8. Paire de garnitures dorsales à échange thermique médicales séparées selon la revendication 7, dans laquelle pour chacune de ladite première garniture dorsale et de ladite seconde garniture dorsale, ladite couche supérieure est définie par une couche souple.

9. Paire de garnitures dorsales à échange thermique médicales séparées selon la revendication 8, dans laquelle pour chacune de ladite première garniture dorsale et de ladite seconde garniture dorsale, ladite couche inférieure définit en outre entièrement une matrice d'ergots dressés, et dans laquelle ladite matrice d'ergots s'étend en travers de ladite pluralité de canaux de fluide.

10. Paire de garnitures dorsales à échange thermique médicales séparées selon la revendication 1, dans laquelle pour chacune de ladite première garniture dorsale et de ladite seconde garniture dorsale, ladite couche conforme comprend :
un premier matériau en suspension dans une matrice définie par un second matériau.

11. Paire de garnitures dorsales à échange thermique médicales séparées selon la revendication 10, dans laquelle pour chacune de ladite première garniture dorsale et de ladite seconde garniture dorsale, ledit premier matériau comprend un liquide et ledit second matériau comprend un polymère.

12. Paire de garnitures dorsales à échange thermique médicales séparées selon la revendication 1, dans laquelle pour chacune de ladite première garniture dorsale et de ladite seconde garniture dorsale, ladite couche conforme comprend un matériau hydrogel.

13. Paire de garnitures dorsales à échange thermique médicales séparées selon la revendication 1, dans laquelle pour chacune de ladite première garniture dorsale et de ladite seconde garniture dorsale, un du premier orifice et du second orifice est situé dans un des premier et second segments de rabat latéral.

14. Agencement comprenant une paire de garnitures dorsales à échange thermique médicales séparées selon l'une quelconque des revendications 1 à 13, et comprenant en outre :
une pompe,
dans lequel le premier orifice et le second orifice de la première garniture dorsale sont conçus pour être interconnectés à la pompe par l'intermédiaire de lignes de tubes interconnectées pour la circulation d'un fluide par la pompe à travers les première et seconde pluralités de canaux de la couche contenant un fluide de la première garniture dorsale ; et,
dans lequel le premier orifice et le second orifice de la seconde garniture dorsale sont conçus pour être interconnectés à la pompe par l'intermédiaire de lignes de tubes interconnectés pour la circulation d'un fluide par la pompe à travers les première et seconde pluralités de canaux de la couche contenant un fluide de la première garniture dorsale.

15. Agencement selon la revendication 14, dans lequel ladite pompe est conçue pour aspirer ledit fluide à travers ladite première garniture dorsale sous une pression négative, et dans lequel ladite pompe est conçue pour aspirer ledit fluide à travers ladite seconde garniture dorsale sous une pression négative.
